(19) Europäisches Patentamt
European Patent Office
Office européen des brevets

(11) EP 4 763 276 A1

(12) EUROPEAN PATENT APPLICATION

(43) Date of publication:
24.06.2026 Bulletin 2026/26

(21) Application number: 24222101.8

(22) Date of filing: 20.12.2024

(51) International Patent Classification (IPC):
A61N 5/10 (2006.01)

(52) Cooperative Patent Classification (CPC):
A61N 5/103; A61N 5/1047

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Siemens Healthineers International AG
6312 Steinhausen (CH)

(72) Inventor: PELTOLA, Jarkko
04300 Tuusula (FI)

(74) Representative: HKW Intellectual Property PartG
mbB
Theresienhöhe 12
80339 München (DE)

(54) **AUTOMATIC TREATMENT FIELD SPLITTING METHOD FOR CONTROLLING TREATMENT TIME IN THE PLAN**

(57) A computer-implemented method, a computer-implemented apparatus, a system and a computer program product for radiation treatment planning are suggested, the method comprising defining a radiation field associated with an initially defined radiation process, automatically splitting the radiation field into a plurality of N sub-fields, with $N \geq 2$ and $n \in \mathbb{N}$, based on a set of metrics, the set of metrics at least comprising a time limitation associated with a duration of a radiation process for at least one of the N sub-fields, and providing a radiation beam configuration for controlling a radiation apparatus adapted to provide the radiation process associated with the split radiation field.

FIG 2B

## Description

The present invention relates to a computer-implemented method, a computer program product, an apparatus and a system for radiation treatment planning.

The increasing number of cancer diagnoses every year increases the demand for modern treatment facilities that are capable of providing an increasing number of patients access to adequate treatment schemes.

A commonly used treatment method for cancer therapy is radiation therapy wherein tumor cells are at least locally irradiated by a suitable radiation beam such as, e.g., X-rays, gamma radiation, protons, heavy ions, etc. A critical aspect of radiation therapy is the radiation dose required to effectively harm malign tumor cells while ensuring that the radiation dose deployed in healthy tissue is minimized (e.g., to avoid the risk of causing carcinogenic cell defects as a result of the radiation process) while keeping the overall duration (e.g., the total time required to deliver a pre-defined radiation dose to a malign tissue of a patient) of the radiation process as low as possible.

These demands are additionally accompanied by the aspect that a patient may move during a radiation process which can cause unwanted effects to the treatment. In some cases, the radiation beam can, e.g., miss the target tumor (at least partially). As a result, healthy tissue may unnecessarily undergo the radiation process with potentially harmful effects and/or a required dose to ensure a treatment of a tumor may not be delivered to the tumor as such but may instead be at least partially distributed in a circumference of the actual malign tissue. Consequently, the possible success of an irradiation procedure may disadvantageously be affected even though a patient may be asked not to move.

In some cases, the unwanted movement of the patient may, e.g., be caused by a breathing of the patient which may cause a (periodic) movement of at least a chest of the patient. This problem may, e.g., be addressed by asking the patient not to breath for a certain period of time which, however, may require that the duration of the radiation process is chosen to be as short as possible.

The treatment planning process is oftentimes based on treatment planning software solutions which may cause the radiation time for a treatment plan to be inadequately controllable for a physician or any other planner (e.g., a member of a medical staff).

In case, breath-holds are required during a treatment of a patient, dedicated controls may be used that may allow to pause a radiation treatment temporarily in case a patient needs to breath. Such a pause may either be set manually or automatically. In case of an automatic pausing, detectors may be used that determine and track the chest movement of a patient and may adapt the radiation process accordingly (e.g., stop the radiation process in case a chest movement is detected).

As a result, the radiation procedure as such may essentially (and sometimes non-deterministically) be split into several fractions as a result of the breathing of the patient which, however, is currently not used for the treatment planning as such. Splitting of an initial radiation field into several static fractions during planning may, however, not adequately consider the physiology of the patient (e.g., a breathing frequency of the patient) during radiation treatment planning or does not provide the option to plan a splitting of a radiation procedure at all as (fixed) time-based intervals may be used directly instead.

Thus, a tailored treatment planning, adapted to the physiology of the patient (e.g., a breathing frequency of the patient) may not be possible in a satisfying manner such that a treatment planning may not always be optimized for the respective patient.

It is one object to provide a treatment planning solution that may be more closely tailored to actual physiology and/or needs of a patient.

According to a first aspect, a computer-implemented method for radiation treatment planning is suggested. The method comprises defining a radiation field associated with an initially defined radiation process, automatically splitting the radiation field into a plurality of N sub-fields, with $N \geq 2$ and $N \in \mathbb{N}$, based on a set of metrics, the set of metrics at least comprising a time limitation associated with a duration of a radiation process for at least one of the N sub-fields and providing a radiation beam configuration for controlling a radiation apparatus adapted to provide the radiation process associated with the split radiation field.

A radiation field (also called radiotherapy field) may be understood as one single, continuous way to produce and modulate radiation originating from a radiation source that may follow a geometric path around the patient. In case a radiation source can be rotated around the patient, i.e. having a rotating gantry, the field may be called an arc field. The radiation field may be associated with a spatial extension of an area in which radiation is transmitted from a gantry to a patient as the gantry is moved.

The defining of an initial radiation field may at least partially be performed by a physician and/or a member of a medical staff (such as, e.g., a radiation physicist or a trained radiation planner who may define the initial radiation field and/or the further splitting of the initial radiation field). In some examples, the physician may only set general guidelines/boundary conditions (e.g., defining a target area in the body of the patient that needs to be exposed to a radiation beam) for the radiation planning without being involved in any technical radiation planning at all. In some cases, the defining of the radiation field may be performed automatically (without a need for an intervention of a physician). In some cases, the defining of the radiation field may be based on a synergy of the defining by a physician and by a respective software tool.

A radiation field associated with a plurality of N sub-fields may be understood as a radiation field that forms a sub-portion of the initially defined radiation field. In other words, a radiation field associated with the N sub-fields may be fully comprised by the initially defined radiation field.

The time limitation may be understood as a temporal upper limit on the duration of a radiation process.

The radiation beam configuration may comprise all data that is required to set the radiation apparatus into a state in which it may provide the radiation process and the N sub-fields as determined as part of the computer-implemented method.

The splitting of the initially defined radiation field into N sub-fields may allow a tailored adaptation of the respective radiation process to the physiology of a patient as the individual N sub-fields obtained as a result of the splitting may be determined such that a respective duration of the radiation process may not exceed a certain upper limit. As a result, a planner may efficiently prepare for a potential movement of the patient before the treatment starts. Therefore, the safety of a radiation process may be increased as the risk that healthy tissue is erroneously irradiated as a result of a movement of the patient may be reduced. What is more, the efficiency of the radiation process may be increased as it may be ensured that the dose deployed in malign tissue indeed arrives in the malign tissue and is not spread about the actual tumor. Even more, since the splitting of the initially defined radiation field is performed automatically, a simplified and faster radiation treatment planning may be facilitated.

Even further, the proposed method may allow a deterministic planning of a radiation treatment where the exact behavior of a radiation apparatus is known beforehand. Compared to a manual splitting of an initially defined radiation field, a more natural and direct control of the splitting may be achieved.

In some examples, the method may be based on using conventional/already finished treatment plans such that a splitting may be based at least in part on information of previously split initially defined radiation fields. Therefore, the splitting strategy is not required to be guessed but may be based at least in part on existing knowledge on an adequate splitting. In some examples, the splitting may be applied to already existing treatment plans that are directed to a single radiation field without any splitting.

Radiation apparatus may be an apparatus capable of providing a radiation beam such as, e.g., X-rays, gamma rays, protons, (charged) heavy ions and/or any other suitable radiation source that may be capable of providing a radiation beam for the radiation of malign tissue. Radiation apparatus may comprise a gantry that may be rotated about a patient to be irradiated who may be placed at a center region of the gantry. In some examples, the radiation device may comprise a collimator (e.g., a multi-leaf collimator) adapted to shape the radiation beam.

According to an embodiment, defining the radiation field may comprise defining an arc field, e.g., a 360° arc field.

An arc field may be understood as being associated with the movement of a gantry of the radiation apparatus that predominately moves along an arc shaped trajectory. An arc may be understood as defined by at least a portion of a circle that is (predominantly) centered about a patient.

In case of a 360° arc field, the gantry may be configured to fully execute a full revolution about a patient during a radiation process.

In some cases, the gantry may be configured to execute a full revolution of the 360° arc field in 20 seconds, 30 seconds, 40 seconds, 50 seconds, 60 seconds, 70 seconds, 80 seconds, 90 seconds or any other suitable duration.

In some examples, the arc field may span less than 360° such as, e.g., 330° to 359°, or 340° to 358°. In some examples, the arc field may span 358°. In some examples, e.g., in breast-planning treatments, an arc field may be provided one-sided and may span an arc field of 180° or 270°.

A radiation arc field may thus be defined by the radiation field supplied to a patient as the gantry of the radiation apparatus is moved along an arc about the patient.

By defining a radiation field that comprises defining an arc field, a trajectory for the gantry may be provided that may efficiently and cost-effectively be realized.

According to a further embodiment, splitting the radiation field may comprise splitting the radiation field into N, e.g., 6 subsequently arranged arc sub-fields, each sub-field extending along a 360°/N arc sub-field angular sequence along a circumferential direction of the 360° arc field.

By splitting the initially defined radiation field into sub-fields that are arranged subsequent to each other at 360°/N arc sub-field angular sequences, the initially defined radiation directions that are associated with the defined initial radiation field are maintained. Moreover, the total radiation dose to be delivered to the malign tissue of a patient may be maintained such that a successful radiation treatment may be ensured.

In some examples, the radiation field may be split into 2, 3, 4, 5, 7, 8, 9, 10 or even a higher number of sub-fields.

By specifically splitting the initially defined radiation field into 6 subsequently arranged sub-fields, a duration of the respective radiation process may be defined that may allow a patient to more easily avoid any movement during a radiation process of one of the arc sub-fields. Therefore, an unwanted movement of the patient may efficiently be suppressed. Consequently, a precise delivery of a radiation dose in malign tissue may be supported.

According to a further embodiment, the method may comprise interrupting the radiation process of the N-1th arc sub-field for a predetermined time interval at a transition from a radiation process associated with the N-1th arc sub-field to a radiation process associated with an Nth arc sub-field for $N \in \mathbb{N}$.

Interrupting the radiation process may, e.g., comprise blocking the radiation beam during the predetermined time interval (e.g., by placing an absorber between a radiation source and the patient) and/or switching a respective radiation source off.

Interrupting the radiation process for a predetermined time interval at a transition between two subsequently arranged sub-fields may allow the patient to move. Therefore, a radiation treatment may more closely be tailored to the physiology of the patient.

According to a further embodiment, the time limitation may be determined by a user-defined time limitation, e.g., by a breath-hold time of a patient.

A user-defined time limitation may be provided by a physician and/or a member of a medical staff and/or a patient.

By allowing a user-defined time limitation, the radiation treatment planning may be more dynamic and flexible and may be adapted more closely to the needs of the patient.

A breath-hold time may be understood as the duration during which the patient may hold his breath (e.g., during which the patient may (actively) stop breathing).

This may allow an adaptation of a radiation process to the natural physiology of the patient by taking a duration into account during which a patient may hold his breath (e.g., for 10 seconds, 15 seconds, 20 seconds, etc.). Therefore, the radiation treatment planning may more closely be tailored to the needs of the patient. What is more, by splitting the initially defined radiation field into N sub-fields, wherein the time limitation is determined by the breath-hold time of the patient, it may efficiently be ensured that the radiation beam delivers its energy to the malign tissue without disadvantageously harming the healthy tissue of the patient as, e.g., an unwanted chest movement of the patient (e.g., as a result of the breathing) may largely be suppressed. Therefore, a more accurate irradiation may be provided to the patient.

According to a further embodiment, the set of metrics may comprise information associated with a control point, e.g., a meterset weight value and/or a predefined gantry angle.

A meterset weight may represent a dose that is to be delivered at a certain control point such that the radiation field may be split into subfields that produce the same amount of dose. The meterset weight may, e.g., be defined as indicating a radiation dose to be delivered at a certain angle of the gantry (e.g., relative to an initial/starting position of the gantry). Additionally or alternatively, the metric may also comprise an indicator that indicates that a meterset weight is equal to zero, i.e., the indicator may indicate that no radiation is to be delivered at a certain control point. The latter may facilitate a transition between two subsequent sub-fields during which transition the radiation beam may be switched off. Additionally or alternatively, the set of metrics may comprise predefined gantry angles (e.g., relative to an initial/starting position of the gantry) at which a splitting of the radiation field should occur.

According to a further embodiment, the radiation field may at least partially be defined by a sequence of subsequently arranged control points along an arc-shaped trajectory surrounding the patient.

Each control point may be associated with a configuration of the gantry used for the respective radiation process.

A control point may comprise one or more of a gantry angle, a couch angle, a collimation angle, multi-leaf collimator positions, a collimation jaw positions, an iso-center and a meterset weight (which tells how much radiation/dose is to be delivered from a previous control point).

It is noted that the trajectories surrounding the patient are not necessarily limited to arc-shaped trajectories only. In general, any trajectory may be implemented along which a radiation dose may be applied to a patient. Such trajectories may, e.g., be obtained by rotating the gantry about the patient and/or by moving/rotating the couch and/or a collimator.

By providing a sequence of control points, the gantry may be provided with information at each operation point during a full or at least partial revolution of the gantry about a patient.

According to a further embodiment, at least two adjacent arc sub-fields may at least partially overlap along a circumferential direction.

An overlap between two adjacent arc-subfields may allow an at least partial overlap of a configuration of the radiation beam associated with a first arc sub-field and of a configuration of the radiation beam associated with a second arc sub-field. As a result, a step-like and sharp transition from a first arc sub-field to a second arc sub-field may efficiently be suppressed.

According to a further embodiment, the method may further comprise smoothing a supplied intensity of a radiation beam associated with the radiation process during a transition from the N-1th arc field to the Nth arc field, with $N \in \mathbb{N}$, by modulating an intensity of the radiation beam.

A smoothing may be understood as a suppression of a sharp transition from the N-1th arc field to the Nth arc field, e.g., by temporally decreasing a radiation beam intensity during an overlap of the respective two adjacently arranged sub-fields. In some examples, the radiation beam intensity may be adapted (based at least in part) in dependence from a current location of the gantry.

The radiation beam intensity may be adapted based on a (predetermined) mathematical relationship between the radiation beam intensity and a position of the gantry. In some examples, the radiation beam intensity may be decreased (linearly) if a transition is approached, may be kept at a lower level as compared to an original radiation beam intensity and may be increased again (linearly) to the originally used radiation beam intensity.

A smoothing may suppress explicit cut points between two adjacent arc fields and may thus support a homogenous and controlled radiation intensity across transition points. Moreover, any sort of hard features in the dose that might appear in reality because of cut points may be minimized.

A 3D dose distribution in the patient is formed by the radiation field and may be linear in nature so that each portion of radiation coming from each gap between two subsequently arranged control points provides a certain dose contribution originating from, e.g., a certain gantry angle to the sum of the delivered radiation dose. The radiation field originating from a certain gantry angle configuration may be associated with a complex configuration how the radiation beam may be shaped (e.g., leaf positions etc.). If said radiation beam is abruptly paused for a while (e.g., between two subsequently arranged control points of subsequently arranged sub-fields), and then resumed again with the same complex radiation beam configuration, hard transitions may arise that may be understood as hard features. More specifically, a hard boundary may not only arise in angle but additionally also in respective leaf positions between an $N\text{-}1^{th}$ and an $N^{th}$ sub-field.

During the pause between two subsequently arranged sub-fields, a patient may (disadvantageously) be given more time to move unfavorable (with respect to a scheduled radiation treatment), e.g. shifting 1 cm left, in respect to the field. Thus, the place where the dose from that angle direction originates is not what may have been planned beforehand and the hard boundary line can be visible in the radiation dose distribution (e.g., in the patient). By smoothing out the transition a distribution of a potential error to a wider region may thus be facilitated.

According to a further embodiment, the splitting may comprise splitting the radiation field such that a sum of the radiation doses associated with each of the N arc sub-fields is equal to a total radiation dose defined for the radiation field.

This may ensure that the total dose to be delivered to malign tissue, as defined for the initially defined radiation field is maintained even though the initially defined radiation field is split into sub-fields. This may ensure a predetermined radiation dose is delivered to a patient while taking advantage of a better adaptation of the radiation process to the physiology of the patient.

Any embodiment of the first aspect may be combined with any embodiment of the first aspect to obtain another embodiment of the first aspect.

According to a second aspect, a computer program product comprising instructions is proposed which, when the program is executed by a computer, cause the computer to carry out the computer-implemented method of the first aspect or any embodiment of the first aspect.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a third aspect, a computer-implemented apparatus for radiation treatment planning is suggested. The computer-implemented apparatus may comprise a defining unit for defining a radiation field associated with an initially defined radiation process, a splitting unit for automatically splitting the radiation field into a plurality of N sub-fields, with $N \geq 2$ and $N \in \mathbb{N}$, based on a set of metrics, the set of metrics at least comprising a time limitation associated with a duration of a radiation process for at least one of the N sub-fields and a providing unit for providing a radiation beam configuration for controlling a radiation apparatus adapted to provide the radiation process associated with the split radiation field.

According to an embodiment, the computer-implemented apparatus may comprise an execution unit for executing the computer-implemented method as outlined above.

According to a further embodiment, the computer-implemented apparatus may further comprise a further execution unit for executing the computer program product as outlined above.

Each of the aforementioned entities, e.g., each of the aforementioned units, may be implemented in hardware and/or in software. If said entity is implemented in hardware, it may be embodied as a device, e.g. as a computer or as a processor or as a part of a system, e.g. a computer system. If said entity is implemented in software it may be embodied as a computer program product, as a function, as a routine, as a program code or as an executable object.

According to a fourth aspect, a system for radiation treatment planning is suggested. The system comprises the computer-implemented apparatus as outlined above, and the computer program product as outlined above.

In one or more examples, the functions described may generally be implemented in hardware, software, firmware, or any combination thereof. If implemented in software, the functions may be stored on or encoded as one or more instructions or code on a computer-readable medium. Computer-readable media includes computer storage media. Storage media may be any available media that can be accessed by a computer. By way of example, and not limitation, such computer-readable media can comprise random-access memory (RAM), read-only memory (ROM), electronically erasable programmable ROM (EEPROM), compact disk (CD) ROM (CD-ROM), or other optical disk storage, magnetic disk storage or other magnetic storage devices, or any other medium that can be used to carry or store desired program code in the form of instructions or data structures and that can be accessed by a computer. Disk and disc, as used herein, includes CD, laser disc, optical disc, digital versatile disc (DVD), and floppy disk where disks usually reproduce data magnetically, while discs reproduce data optically with lasers. Combinations of the above should also be included within the scope of computer-readable media.

The embodiments and features described with reference to the disclosed apparatus apply *mutatis mutandis* to the disclosed method and vice versa.

Further possible implementations or alternative solutions also encompass combinations - that are not explicitly mentioned herein - of features described above or below with regard to the embodiments. The person skilled in the art may also add individual or isolated aspects and features to the most basic form of the apparatus and/ or the method.

Further embodiments, features and advantages will become apparent from the subsequent description and dependent claims, taken in conjunction with the accompanying drawings, in which:

> Fig. 1 shows an exemplary system implementing a computer-implemented apparatus according to embodiments;

> Figs. 2A and 2B show an exemplary radiation treatment planning procedure;

> Figs. 3A-3C show exemplary configuration parameters that may be associated with a control point;

> Fig. 4 shows a block diagram associated with an exemplary computer-implemented method for radiation treatment planning;

> Fig. 5. shows an exemplary computer-implemented apparatus for radiation treatment planning; and

> Fig. 6 shows an exemplary system for radiation treatment planning.

In the Figures, like reference numerals designate like or functionally equivalent elements, unless otherwise indicated.

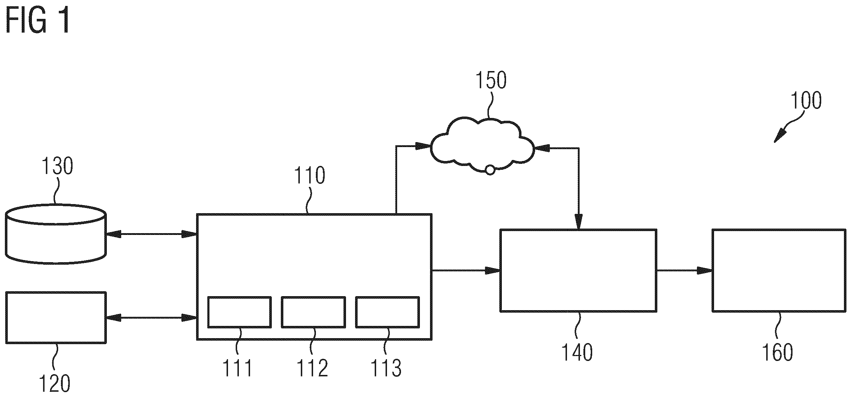

Fig. 1 shows an exemplary system 100 implementing a computer-implemented apparatus 110 according to embodiments.

System 100 comprises a computer-implemented apparatus 110 as described elsewhere herein. The computer-implemented apparatus 110 comprises a defining unit 111 as described elsewhere herein. The computer-implemented apparatus may comprise a splitting unit 112 for automatically splitting a radiation field as described elsewhere herein. Moreover, the computer-implemented apparatus 110 may further comprise a providing unit 113 for providing a radiation beam configuration for controlling a radiation apparatus 140 as described elsewhere herein.

Computer-implemented apparatus 110 may be in (bidirectional) communication (e.g., wired and/or wireless) with a user interface 120. User Interface 120 may be provided as a (touch-sensitive) screen, a speech-to-text conversion unit, a handheld device (e.g., a tablet, a notebook, a mobile phone, etc.) which may be adapted to receive a user input received from a user of the system 100. Additionally or alternatively, the user interface 120 may be configured to receive one or more commands from the computer-implemented apparatus 110 and may be adapted to provide the received commands as information to the user of the system 100. In some examples, the user interface 120 may thus display a prompt to the user of the system 100 requesting information associated with a treatment planning process (as described elsewhere herein).

Computer-implemented apparatus 110 may be in (bidirectional) communication (e.g., wired and/or wireless) with a remote entity 130. Remote entity 130 may act as a remote storage facility, e.g., a database configured to store information that may be associated with a radiation treatment planning according to aspects as discussed above. That information may, e.g., comprise information associated with a previously performed radiation treatment planning, CT and/or X-ray scans associated with a patient to be irradiated. In some examples, remote entity 130 may be configured to store a (hospital) patient file and/or any other patient-related data that may be useful for a radiation treatment planning. In some examples, remote entity 130 may store one or more previously generated and/or used radiation treatment plans.

Computer-implemented apparatus 110 may further be in communication with the radiation apparatus 140. Radiation apparatus 140 may be an apparatus capable of providing a radiation beam such as, e.g., X-rays, gamma rays, protons, (charged) heavy ions and/or any other suitable radiation source that may be capable of providing a radiation beam for the radiation of malign tissue. Radiation apparatus 140 may comprise a gantry that may be rotated about a patient to be irradiated who may, e.g., be placed at a center region of the gantry. In some examples, the radiation device may comprise a collimator (e.g., a multi-leaf collimator) adapted to shape the radiation beam.

Radiation apparatus 140 may be configured to perform a radiation process in accordance with a configuration received from the computer-implemented apparatus 110. In some examples, the configuration may comprise one or more control points for controlling a movement/rotation of the gantry as described elsewhere herein.

Radiation apparatus 140 may generate a radiation (beam) in accordance with the received configuration and may be configured to direct the generated radiation beam onto a patient 160 to be irradiated.

In some examples, computer-implemented device 110 may additionally or alternatively be in communication with a cloud service 150 that may be hosted at a remote server and/or a hospital server and/or at any other suitable entity. Cloud service 150 may be in (bidirectional) communication with the radiation apparatus 140. Cloud service 150 may be configured to receive a configuration for radiation apparatus 140 as provided by the computer-implemented device 110 and may at least temporarily store the configuration, e.g., in a database. Cloud service 150 may be accessible, e.g., by a physician by means of an internet connection and/or a local area network.

Cloud service 150 may be configured to relay the received configuration to radiation apparatus 150.

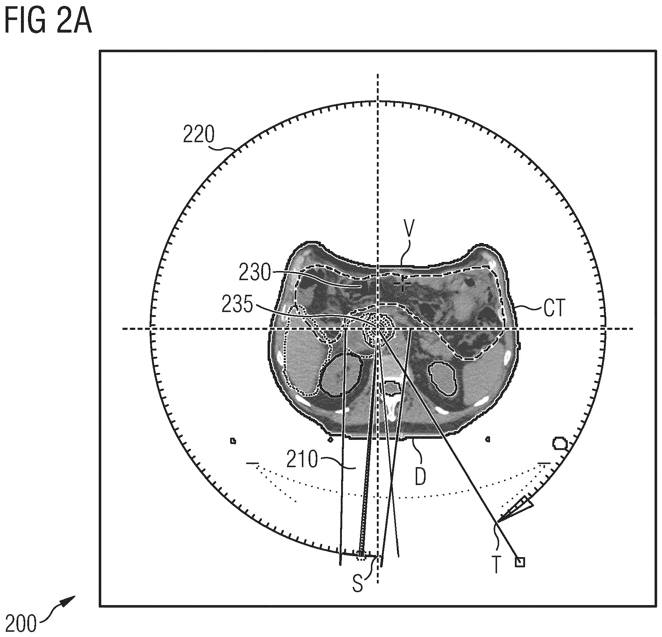

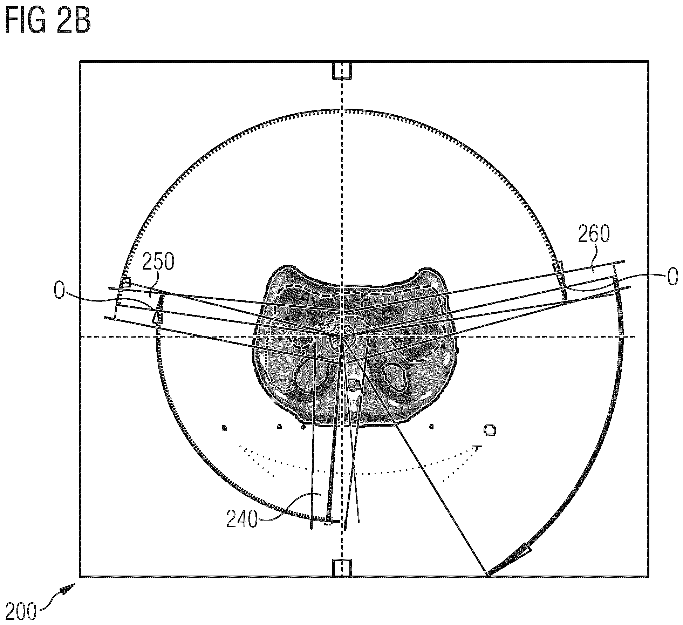

Figs. 2A and 2B show an exemplary radiation treatment planning procedure 200 according to some embodiments.

Fig. 2A shows an exemplary defining of a radiation field 210 associated with an initially defined radiation process.

Radiation field 210 may originate from a gantry wherein a movement of the gantry may be defined by a sequence of subsequently arranged control points 220 where the control points 220 are, e.g., arranged along an arc-shaped trajectory surrounding a patient 230. The resulting radiation field may also be referred to as an arc-field.

The movement of the gantry may be defined by a start point S, may follow the subsequently arranged control points 220 and may terminate at end point T. In some examples, the gantry may perform a full revolution about the patient 230. In that case, the gantry may define a 360° angular segment from the start point S of the gantry to the end point T of the gantry along its arc shaped trajectory.

In some examples, the gantry may only undergo a fraction of a full revolution about the patient 230. That is, in some examples, the gantry may define a 360°/N, with

$N \in \mathbb{N}$ , angular segment as it moves along its arc shaped trajectory.

Fig. 2A further depicts an exemplary CT image CT taken along a transverse cross section through the patient 230 wherein the transverse cross section shows the body of the patient as extending from a ventricular side V of the patient 230 to a dorsal side D of the patient 230.

Patient 230 may suffer from a tumor 235 which may be understood as malign tissue as described elsewhere herein.

Radiation field 210 may be adapted such that tumor 235 lies within the radiation field 210 and such that a desired radiation dose may be deployed in the tumor 235.

Fig. 2B shows an exemplary radiation treatment process 200 according to some embodiments.

According to some embodiments, the initially defined radiation field 210 is split into three sub-fields 240, 250 and 260. It is noted that splitting the originally defined radiation field 210 into three sub-fields is only an example and that a splitting of the initially defined radiation field 210 into a different number of sub-fields is equally possible (e.g. into any number of 2 to twenty sub-fields, e.g., into 3 to 15 sub-fields, into 4 to 10 sub-fields or into 5 to 8 sub-fields).

Each of the sub-fields 240, 250 and 260 may, in some examples, be arc-shaped and defined by a set of control points 220. Each of the sub-fields 240, 250 and 260 may be understood in analogy to the radiation field 210 but spanning a smaller angular section as compared to the radiation field 210. In the depicted example of splitting the initially defined radiation field into three sub-fields, each of the sub-fields may be configured such that it spans across a 360°/3 = 120° angular sequence.

In some examples, each of the sub-fields 240, 250 and 260 may start where another sub-field ends.

In some examples, at least two of the sub-fields 240, 250 and 260 may at least partially overlap thus defining an overlapping region O. In some examples, adjacently arranged sub-fields 240 and 250 may overlap in overlapping region O. Additionally or alternatively, adjacently arranged sub-fields 250 and 260 may at least partially overlap in overlapping region O. As a result of the overlapping region O, a smoothing of the supplied intensity of a radiation beam may be ensured. This may, e.g., be achieved by ramping up/down the intensity of the radiation beam accordingly (e.g., by varying a monitoring unit (MU) and/or a meterset weight).

It is appreciated that even though the control points 220 associated with a respective sub-field 240, 250 and 260 are depicted as being geometrically displaced relative to each other, they may nevertheless be assigned to the same gantry arranged at a constant distance from the center of the arc.

If a sequence of subsequently arranged control points 220 is known, a duration of a radiation process associated with said control points 220 may be estimated. Therefore, even if a detailed knowledge of the radiation apparatus used is unknown, the control points 220 may nevertheless still allow a time estimate of the duration of a respective radiation process, e.g., by using a linear model. Each control point 220 represents a configuration of the radiation apparatus and it may be assumed that a transition between two control points 220 follows a linear change in the mechanical axis. Since each mechanical axis has a maximum speed, one can estimate how much time is being spent when going through the control points in the respective radiation field. If it is desired to split an initially defined radiation field into smaller sub-fields which have a time limitation, the splitting may be based on the aforementioned estimation of the expected treatment time.

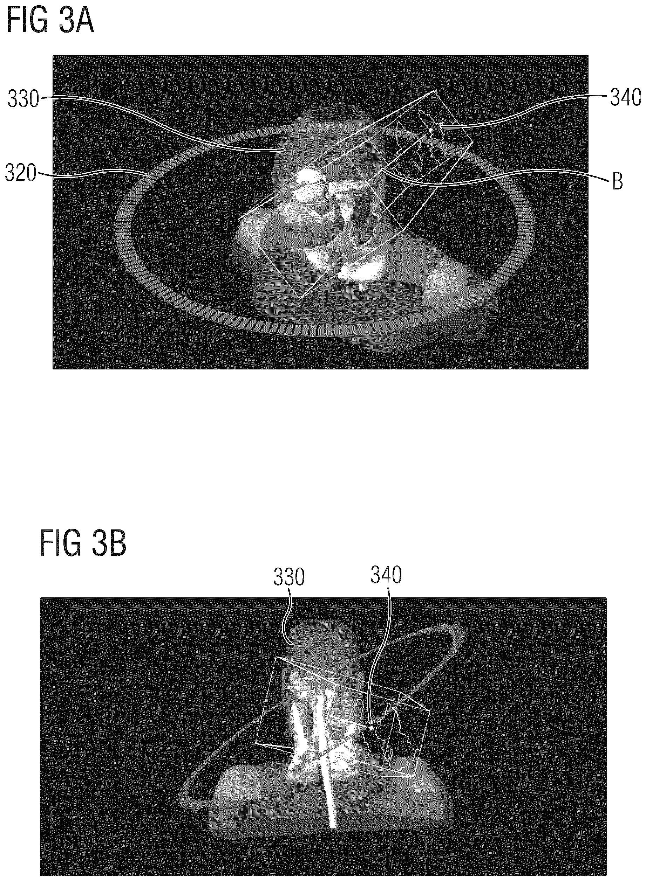

Figs. 3A-3C show exemplary configuration parameters that may be associated with a control point (e.g., control point 220).

Fig. 3A shows a first parameter that may be comprised by a control point 320 and which may configure a gantry angle, i.e., an angle of the gantry relative to an initial start position. This gantry angle may define a direction from which a radiation beam B may be incident onto a patient 330. Each of the control points 320 shown in Fig. 3A as segments may be associated with a respective different gantry angle, e.g., referenced relative to an initial starting position of the gantry prior to a gantry movement.

The configuration of gantry angles may be associated with an initially defined radiation field and/or may be associated with a split initially defined radiation field (e.g., a plurality of sub-fields).

In the example shown in Fig. 3A, the radiation beam B may originate from an upper-left position and may be collimated by means of a multi-leaf collimator 340 that is arranged subsequently to the radiation source (not shown in Fig. 3A) and preceding the patient 330.

Fig. 3B shows a second parameter that may be comprised by a control point 320, notably a couch angle. A couch angle may be understood as an angle of a bed, on which the patient 330 may be placed during a radiation process, relative to a reference point/reference axis associated with the radiation apparatus. In some examples, the couch angle may be defined according to the (standardized) IEC 6121 coordinate system.

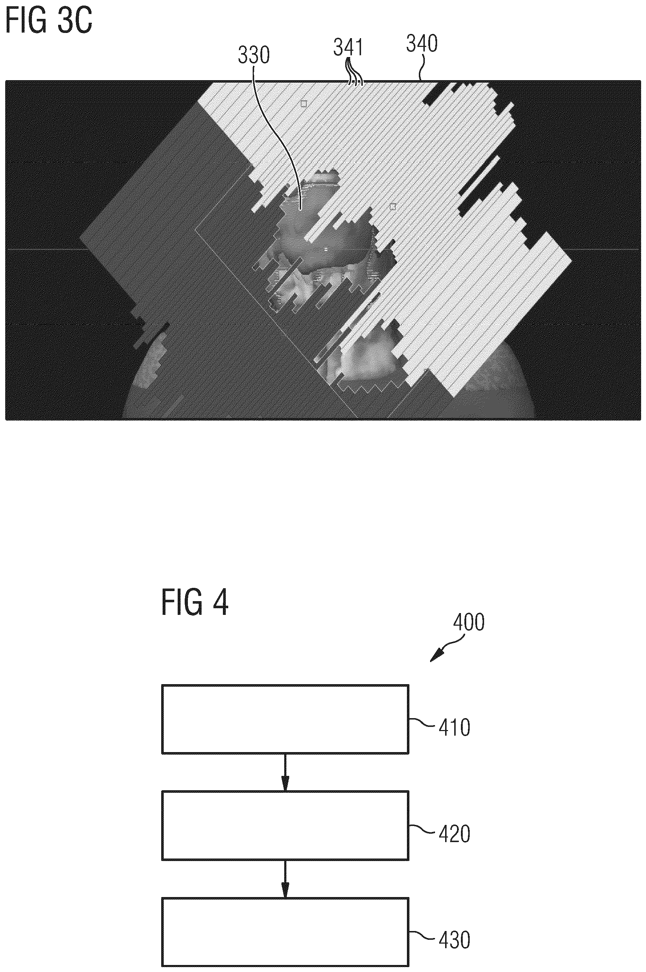

Fig. 3C shows a third parameter that may be comprised by a control point 320, notably a configuration of a multi-leaf collimator 340. A multi-leaf collimator 340 may be provided as a collimator or beam-limiting device that is made of individual leaves 341 of a high atomic numbered material, usually tungsten, that can move independently in and out of the path of a radiotherapy beam in order to shape it and vary its intensity. Multi-leaf collimators 340 may be used in external beam radiotherapy to provide conformal shaping of beams. Specifically, conformal radiotherapy and Intensity Modulated Radiation Therapy (IMRT) can be delivered using multi-leave collimators 340.

The configuration for the multi-leaf collimator 340 may comprise information on an orientation of the multiple leaves 341, such as, e.g., a certain distance a leave is required to move out of a rest position towards the trajectory of the radiation beam B to at least partially block the incident radiation beam B. A combination of multiple leaves 341 may thus allow a shaping of a radiation beam B prior to an interaction of the radiation beam with the patient 330. This may suppress unwanted radiation of healthy tissue that may surround malign tissue that is to be irradiated.

Fig. 4 shows an exemplary computer-implemented method 400 for radiation treatment planning according to some embodiments.

In step 410, a radiation field associated with an initially defined radiation process is defined.

In step 420, an automatically splitting of the radiation field into a plurality of N sub-fields, with $N \geq 2$ and $N \in \mathbb{N}$, based on a set of metrics, the set of metrics at least comprising a time limitation associated with a duration of a radiation process for at least one of the N sub-fields is performed.

In step 430, a radiation beam configuration for controlling a radiation apparatus adapted to provide the radiation process associated with the split radiation field is provided.

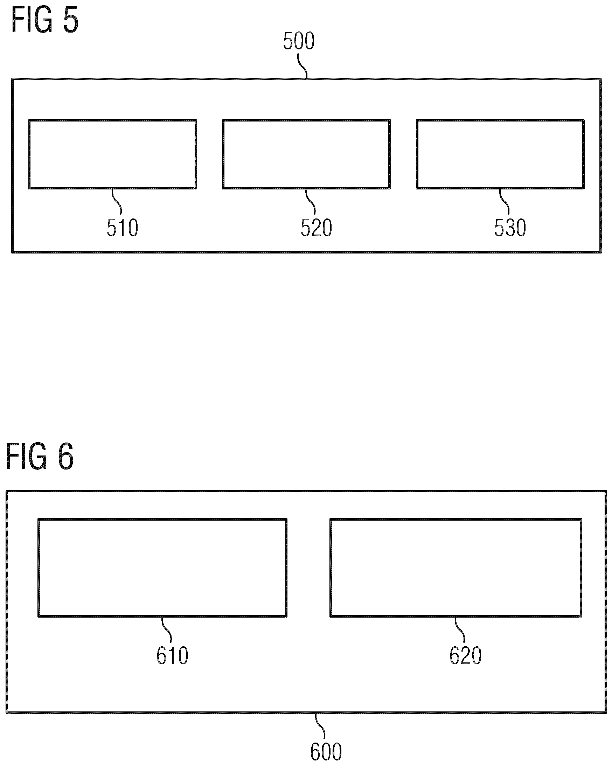

Fig. 5 shows an exemplary computer-implemented apparatus 500 for radiation treatment planning according to some embodiments. The apparatus 500 includes a defining unit 510, a splitting unit 520 and a providing unit 530.

Defining unit 510 may be configured for defining a radiation field associated with an initially defined radiation process.

Splitting unit 520 may be configured for automatically splitting the radiation field into a plurality of N sub-fields, with $N \geq 2$ and $N \in \mathbb{N}$, based on a set of metrics, the set of metrics at least comprising a time limitation associated with a duration of a radiation process for at least one of the N sub-fields.

Providing unit 530 may be configured for providing a radiation beam configuration for controlling a radiation apparatus adapted to provide the radiation process associated with the split radiation field.

Fig. 6 shows an exemplary system 600 for radiation treatment planning according to some embodiments. The system 600 includes a computer-implemented apparatus 610 and a computer program product 620.

Computer-implemented apparatus 610 may be configured as described elsewhere herein.

Computer program product 620 may be configured as described elsewhere herein.

Although the above has been described in accordance with preferred embodiments, it is obvious for the person skilled in the art that modifications are possible in all embodiments.

Independent of the grammatical term usage, individuals with male, female or other gender identities are included within the term.

List of Reference

| 100 | system |
|-----|--------|
| 110 | computer-implemented apparatus |
| 111 | defining unit |
| 112 | splitting unit |
| 113 | providing unit |
| 120 | user interface |

| | |
|---|---|
| 130 | remote entity |
| 140 | radiation apparatus |
| 150 | cloud service |
| 160 | patient |
| 200 | radiation treatment planning procedure |
| 210 | radiation field |
| 220 | control point |
| 230 | patient |
| 235 | tumor |
| 240 | sub-field |
| 250 | sub-field |
| 260 | sub-field |
| 320 | control point |
| 330 | patient |
| 340 | multi-leaf collimator |
| 341 | leaves |
| 400 | computer-implemented method |
| 410 | step |
| 420 | step |
| 430 | step |
| 500 | computer-implemented apparatus |
| 510 | defining unit |
| 520 | splitting unit |
| 530 | providing unit |
| 600 | exemplary system |
| 610 | computer-implemented apparatus |
| 620 | computer program product |
| B | radiation beam |
| | |
| CT | CT image |
| D | dorsal |
| O | overlapping region |
| S | start point |
| T | end point |
| V | ventral |

## Claims

1. A computer-implemented method (400) for radiation treatment planning, comprising:

   defining (410) a radiation field (210) associated with an initially defined radiation process; automatically splitting (420) the radiation field (210) into a plurality of N sub-fields (240, 250, 260), with $N \geq 2$ and $N \in \mathbb{N}$, based on a set of metrics, the set of metrics at least comprising a time limitation associated with a duration of a radiation process for at least one of the N sub-fields (240, 250, 260); and providing (430) a radiation beam (B) configuration for controlling a radiation apparatus (140) adapted to provide the radiation process associated with the split radiation field.

2. The computer-implemented method according to claim 1,

wherein defining (410) the radiation field comprises defining an arc field.

3. The computer-implemented method according to claim 1 or 2,
   wherein splitting (420) the radiation field (210) comprises splitting the radiation field (210) into N subsequently arranged arc sub-fields (240, 250, 260), each sub-field (240, 250, 260) extending along a 360°/N arc sub-field (240, 250, 260) angular sequence along a circumferential direction of the 360° arc field.

4. The computer-implemented method according to one of claims 1-3, further comprising:
   interrupting a radiation process of the N-1th arc sub-field for a predetermined time interval at a transition from the radiation process associated with the N-1th arc sub-field to a radiation process associated with an Nth arc sub-field.

5. The computer-implemented method according to one of claims 1-4,
   wherein the time limitation is determined by a user-defined time limitation.

6. The computer-implemented method according to one of claims 1-5,
   wherein the set of metrics comprises information associated with a control point (220).

7. The computer-implemented method according to one of claims 1-6,
   wherein the radiation field (210, 240, 250, 260) is at least partially defined by a sequence of subsequently arranged control points (220) along an arc-shaped trajectory surrounding the patient (230).

8. The computer-implemented method according to one of claims 1-7,
   wherein at least two adjacent arc sub-fields (240, 250, 260) at least partially overlap along a circumferential direction.

9. The computer-implemented method according to claim 8, further comprising:
   smoothing a supplied intensity of a radiation beam (B) associated with the radiation process during a transition from the N-1th arc field to the Nth arc field, by modulating an intensity of the radiation beam (B).

10. The computer-implemented method according to one of claims 1-9,
    wherein the splitting (420) comprises splitting the radiation field (210) such that a sum of the radiation doses associated with each of the N arc sub-fields (240, 250, 260) is equal to a total radiation dose defined for the radiation field (210).

11. A computer program product comprising instructions which, when the program is executed by a computer, cause the computer to carry out the computer-implemented method of one of the claims 1-10.

12. A computer-implemented apparatus (110, 500) for radiation treatment planning, comprising:

    a defining unit (111, 510) for defining a radiation field (210) associated with an initially defined radiation process;
    a splitting unit (112, 520) for automatically splitting the radiation field (210) into a plurality of N sub-fields (240, 250, 260), with $N \geq 2$ and

    $N \in \mathbb{N}$, based on a set of metrics, the set of metrics at least comprising a time limitation associated with a duration of a radiation process for at least one of the N sub-fields (240, 250, 260); and
    a providing unit (113, 530) for providing a radiation configuration for controlling a radiation apparatus adapted to provide the radiation process associated with the split radiation field.

13. The computer-implemented apparatus according to claim 12, further comprising:
    an execution unit for executing the computer-implemented method of one of the claims 1-10.

14. The computer-implemented apparatus according to claim 12 or 13, further comprising:
    a further execution unit for executing the computer program product of claim 11.

15. A system (600) for radiation treatment planning, comprising:

    the computer-implemented apparatus (610) according to one of claims 12-14; and
    the computer program product (620) according to claim 11.

FIG 1

EP 4 763 276 A1

# FIG 2A

## FIG 2B

## FIG 3A

## FIG 3B

FIG 3C

FIG 4

FIG 5

500

510  520  530

FIG 6

610  620

600

## EUROPEAN SEARCH REPORT

**Application Number**

EP 24 22 2101

### DOCUMENTS CONSIDERED TO BE RELEVANT

| Category | Citation of document with indication, where appropriate, of relevant passages | Relevant to claim |
|---|---|---|
| A | US 2013/193351 A1 (CHENG JASON CHIA-HSIEN [TW] ET AL) 1 August 2013 (2013-08-01) * paragraphs [0098] - [0100], [0130] * ----- | 1-15 |
| A | US 2021/353963 A1 (ZHOU JINGJIE [US] ET AL) 18 November 2021 (2021-11-18) * claims 1-9 * ----- | 1-15 |

**CLASSIFICATION OF THE APPLICATION (IPC)**

INV.
A61N5/10

**TECHNICAL FIELDS SEARCHED (IPC)**

A61N

The present search report has been drawn up for all claims

| Place of search | Date of completion of the search | Examiner |
|---|---|---|
| Munich | 9 May 2025 | Kajzar, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 2101

This annex lists the patent family members relating to the patent documents cited in  the above-mentioned European search report.
The members are as contained in the European Patent Office EDP file on
The European Patent Office is in no way liable for these particulars which are merely given for the purpose of information.

09-05-2025

| Patent document cited in search report | | Publication date | Patent family member(s) | | Publication date |
|---|---|---|---|---|---|
| US 2013193351 | A1 | 01-08-2013 | TW | 201345581 A | 16-11-2013 |
|  |  |  | US | 2013193351 A1 | 01-08-2013 |
| US 2021353963 | A1 | 18-11-2021 | CN | 110740783 A | 31-01-2020 |
|  |  |  | US | 2019336793 A1 | 07-11-2019 |
|  |  |  | US | 2021353963 A1 | 18-11-2021 |
|  |  |  | WO | 2019210456 A1 | 07-11-2019 |

EPO FORM P0459

For more details about this annex : see Official Journal of the European Patent Office, No. 12/82